Europäisches Patentamt

European Patent Office

Office européen des brevets

(19)

(11) Veröffentlichungsnummer: **0 052 827**
**B1**

(12) # EUROPÄISCHE PATENTSCHRIFT

(45) Veröffentlichungstag der Patentschrift:
**19.09.84**

(21) Anmeldenummer: **81109587.6**

(22) Anmeldetag: **10.11.81**

(51) Int. Cl.³: **A 61 K 35/14, A 61 K 35/16**

(54) **Verfahren zur Herstellung von Blutgerinnungsfaktoren und danach hergestellte Präparation der Faktoren II und VII.**

(30) Priorität: **21.11.80 DE 3043857**

(43) Veröffentlichungstag der Anmeldung:
**02.06.82 Patentblatt 82/22**

(45) Bekanntmachung des Hinweises auf die Patenterteilung:
**19.09.84 Patentblatt 84/38**

(84) Benannte Vertragsstaaten:
**AT BE CH DE FR GB IT LI LU NL SE**

(56) Entgegenhaltungen:
**EP - A - 0 018 561**

**CHEMICAL ABSTRACTS, Band 66, Nr. 3, 16. Januar 1967, Seite 881, Nr. 9221p, Columbus, Ohio, USA**

(73) Patentinhaber: **BEHRINGWERKE AKTIENGESELLSCHAFT, Postfach 1140, D-3550 Marburg/Lahn (DE)**

(72) Erfinder: **Schwinn, Horst, Dr., Stümpelstal 27, D-3550 Marburg/Lahn (DE)**
Erfinder: **Heimburger, Norbert, Prof. Dr., Sonnenhang 10, D-3550 Marburg/Lahn (DE)**
Erfinder: **Kumpe, Gerhardt, Perbaler Weg 11, D-3552 Wetter (DE)**
Erfinder: **Preis, Hans Martin, Am Wäldchen 9, D-3550 Marburg/Lahn (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

## Beschreibung

Die Erfindung betrifft ein Verfahren zur Herstellung einer praktisch hepatitissicheren Präparation der Blutgerinnungsfaktoren II (Prothrombin) und/oder VII durch Erwärmen, gegebenenfalls in Gegenwart einer Aminosäure und/oder eines Saccharids oder Zuckeralkohols, in Gegenwart eines Chelatbildners.

Die Blutgerinnung ist ein komplexer, in Stufen ablaufender Vorgang, der durch verschiedene physiologische wie pathologische Ursachen ausgelöst wird und dessen Ablauf von etwa 20 fördernden und hemmenden Faktoren abhängt. Durch Verminderung oder Vermehrung dieser Blutgerinnungsfaktoren treten Störungen der Blutgerinnung auf, die sich teilweise als Krankheiten manifestieren.

So führt zum Beispiel eine Erkrankung der Leber mit verminderter Syntheseleistung dieses Organs zu einem Abfall des Plasma-Prothrombin-Spiegels ( = Faktor-II-Spiegel) und des Plasma-Proconvertin-Spiegels ( = Faktor-VII-Spiegel), ein Vorgang, der zu spontanen, lebensbedrohenden Blutungen führen kann. Faktor-II/VII-Konzentrate sind hier das Mittel der Wahl für eine sofort wirkende Therapie.

Ein Faktor-II-Präparat kann nach der Methode von Soulier et al., Thrombos. Diath. Haemorrh. Suppl. 35, 61 (1969) hergestellt werden.

Ein solches Präparat ist nicht frei vom Risiko einer Hepatitisübertragung. Albumin gilt als hepatitissicher, wenn es in Gegenwart von Stabilisatoren 10 Stunden bei 60° C in wäßriger Lösung erhitzt wird (Gellis, S.S. et al., J. Clin. Invest. (1948) 27, 239). Es ist deshalb anzunehmen, daß ein in Gegenwart geeigneter Stabilisatoren erhitztes Faktor-II/VII-Konzentrat ebenfalls hepatitissicher ist.

In der deutschen Offenlegungsschrift 2 916 711 ist ein Verfahren zur Stabilisierung von Gerinnungsfaktoren in wäßriger Lösung gegen Wärme durch Zusatz einer Aminosäure und eines Mono- oder Oligosaccharids oder Zuckeralkohols beschrieben.

Doch kann auch auf diese Weise ein beträchtlicher Ausbeuteverlust an Faktor II nicht verhindert werden (Beispiel 4 der DE-OS). Die Faktoren VII, IX und X, die in einem Faktor-II-Konzentrat üblicherweise enthalten und für dessen Wirksamkeit wichtig sind, werden unter den beschriebenen Bedingungen vollständig inaktiviert.

Es bestand weiterhin die Aufgabe, ein Verfahren zur Stabilisierung wäßriger Faktor-II-Lösungen gegen Wärme zu finden, um die Aktivitätsverluste geringer zu halten.

Überraschend wurde nun gefunden, daß eine wäßrige Lösung von Faktor II und/oder Faktor VII durch Zusatz eines Chelatbildners wie Ethylendiamino-Tetraessigsäure gegen Wärme stabilisiert werden kann. Für Faktor VII war bisher kein Verfahren zur Stabilisierung gegen eine Inaktivierung durch Wärme bekannt.

Gegenstand der Erfindung ist ein Verfahren zur Herstellung einer praktisch hepatitissicheren Präparation der Blutgerinnungsfaktoren II und/oder VII durch Erwärmen, gegebenenfalls in Gegenwart einer Aminosäure und/oder eines Saccharids oder Zuckeralkohols, dadurch gekennzeichnet, daß in Gegenwart eines Chelatbildners erwärmt wird.

Solche Chelatbildner sind beispielsweise:

Ethylendiamino-tetraessigsäure (EDTA)
Ethylenglycol-bis-(2-aminoethylether)-tetraessigsäure (EGTA)
Diaminocyclohexan-tetraessigsäure (CDTA),
Diaminopropan-tetraessigsäure,
Diamino-2-propanol-tetraessigsäure und
Nitrilotriessigsäure
sowie deren lösliche Alkali-Salze.

Bevorzugt werden die Natriumsalze von Ethylendiaminotetraessigsäure (EDTA) und Ethylenglycol-bis-(2-aminoethylenether)-tetraessigsäure (EGTA) verwendet.

In Anwesenheit solcher Chelatbildner kann die wäßrige Lösung der Gerinnungsfaktoren so lange erhitzt werden, daß eine Übertragung von Hepatitis-Erregern nach dem Stand des heutigen Wissens praktisch auszuschließen ist. Dies gilt vor allem in Verbindung mit Fällungsverfahren, bei denen der Wirkstoff im Überstand bleibt und die Hepatitisviren zusammen mit dem unlöslichen Niederschlag abgetrennt werden können. Eine Präparation, die mindestens 10 Stunden bei ca. 60° C in wäßriger Lösung gehalten wurde, gilt heute als praktisch hepatitissicher, insbesondere, wenn von humanem Gewebe ausgegangen wird, in dem nach einem Test der dritten Generation Hepatitisviren nicht nachgewiesen werden können.

Eine besonders bevorzugte Ausführungsform der Erfindung ist dadurch gekennzeichnet, daß eine Faktor II und/oder Faktor VII enthaltende Lösung, vorzugsweise eine Plasma- oder Plazentafraktion, mit 0,01 bis 0,3 mol/l eines der oben angeführten Chelatbildner, vorzugsweise 0,05 bis 0,3 mol/l des Na-Salzes von Ethylendiamino-Tetraessigsäure und gegebenenfalls mit 1,0 bis 3,0 mol/l mindestens einer der Aminosäuren Glycin, alpha- oder $\beta$-Alanin, Hydroxyprolin, Prolin, Glutamin, alpha-, beta- oder gamma-Aminobuttersäure, vorzugsweise aber Glycin und 20 bis 60% w/w, Mono-, Oligosacchariden oder Zuckeralkoholen, vorzugsweise 1,0 bis 3,0 mol/l Glycin und 20 bis 60% w/w Saccharose, versetzt, auf eine Temperatur zwischen 30° C und 100° C, vorzugsweise 60° C bis 100° C, erhitzt und 1 Minute bis 48 Stunden, vorzugsweise etwa 10 Stunden, bei dieser Temperatur gehalten wird, wobei die kürzeste Zeit der höchsten Temperatur zuzuordnen ist und umgekehrt. Um eine maximale Ausbeute zu erreichen, muß der pH-Wert spezifisch auf die einzelnen in der Lösung vorhandenen Gerinnungsfaktoren abgestimmt werden. Allgemein ist ein pH-Wert in den Grenzen zwischen 6,5 und 8,0 einzuhalten. Es wird eine praktisch hepatitissichere Präparation von Faktor II und/oder Faktor VII erhalten.

Abhängig von der Löslichkeit des Chelatbildners, der Aminosäure bzw. des Kohlenhydrates kann der Wert von 0,3 und 3,0 mol/l beziehungsweise 60% w/w zu höheren Konzentrationen erweitert werden, wenn der Chelatbildner, die Aminosäure beziehungsweise das Kohlenhydrat bei der gewünschten Temperatur eine entsprechend höhere Löslichkeit aufweisen. Die Temperaturbehandlung kann auch in mehreren aufeinanderfolgenden Schritten durchgeführt werden.

Mit der bevorzugt verwendeten Kombination des Dinatriumsalzes der Ethylendiamino-Tetraessigsäure mit Glycin und Saccharose wird unter folgenden Bedingungen ein durch Erhitzen hepatitissicheres Präparat erzielt:

10 bis 20 Stunden Erhitzen auf 60 bis 70°C in Gegenwart von EDTA-Na$_2$ in einer Konzentration von 0,05 bis 0,3 mol/l, von Saccharose in einer Konzentration von 40 bis 60% w/w und Glycin in einer Konzentration von 1,0 bis 2,5 mol/l, bei einem pH-Wert von 6,8 bis 8,0.

Das erfindungsgemäße Verfahren ist durch den Zusatz von EDTA-Na$_2$ dem von DE-OS 2 916 711 überlegen. Wie die Tabelle zeigt, wird die Stabilisierung von Faktor II erheblich verbessert, die von Faktor VII überhaupt erst ermöglicht.

Tabelle

Einfluß von EDTA auf die Stabilität der Gerinnungsfaktoren

| Stabilisatoren | | Gerinnungsfaktor (E/ml) | | | |
| --- | --- | --- | --- | --- | --- |
| | | II Erhitzen | | VII Erhitzen | |
| | | vor | nach | vor | nach |
| Saccharose | 60% w/w | 40 | 4 | 20 | 0 |
| Glycin | 2 mol/l | | | | |
| EDTA | 0,1 mol/l | 40 | 38 | 20 | 18 |
| Saccharose | 60% w/w | | | | |
| Glycin | 2 ml/l | | | | |

Die Rückgewinnung und Reinigung der Gerinnungsfaktoren aus der erhitzten Lösung kann durch Fällen mit Ammoniumsulfat, 30—45% w/v, und Adsorption des Überstandes an Ca-Phosphat, 0,4 bis 1,0% w/v, erfolgen.

Zweckmäßigerweise geht man von Fraktionen aus, in denen der zu stabilisierende Faktor gemäß dem zitierten Verfahren angereichert ist.

Die Kontrolle der Maßnahmen zur Anreicherung und Reinigung von Faktor II oder VII sind dem Fachmann durch die Kenntnis von Bestimmungsmethoden für die betreffenden Substanzen geläufig. Unter Verwendung dieser Kontrollmethoden können die Verfahrensbedingungen unter dem Gesichtspunkt einer befriedigenden Ausbeute und einer befriedigenden Reinheit des Produktes gelenkt werden.

Um zu einem hepatitissicheren Faktor-II- und/oder -VII-Konzentrat zu gelangen, geht man von einer Fraktion aus, wie sie beispielsweise nach dem Verfahren von Soulier et al., Thrombosis Diath. Haemorrh. Suppl. 35, 61 (1969) erhalten wird. Dazu adsorbiert man Plasma, das aus einem mit 0,01 mol/l EDTA anticoaguliertem Blut gewonnen wurde, mit Ca-Phosphat und zentrifugiert ab. Dabei werden die Faktoren quantitativ an das Adsorbens gebunden und können durch mehrfaches Eluieren mit 0,2 mol/l Tri-Natriumcitrat wieder gewonnen werden. Die vereinigten Eluate werden durch kombinierte Alkohol- und Essigsäurefällungen bei Temperaturen von —8°C bis +4°C weiter gereinigt. Dabei werden die Faktoren gleichzeitig konzentriert.

Man nimmt das Konzentrat in einem geeigneten Puffer, vorteilhaft Kochsalz/Natriumcitrat mit einer Konzentration von 0,06 bzw. 0,02 mol/l bei pH 7,6 auf und bestimmt die Aktivität der Faktoren.

Die Aktivitätsbestimmungen sind dem Fachmann bekannt. Für Faktor II kann sie zum Beispiel nach der Methode von Koller, F. et. al., Dtsch. med. Wschr. 81, 516 (1956), erfolgen. Dazu wird ein Teil, zum Beispiel 0,1 ml Faktor-II-Mangelplasma, und ein Teil verdünntes Normalplasma vermischt. Diese Mischung wird 30 Minuten bei +37°C gehalten. Danach setzt man zwei Teile calciumhaltiges Thromboplastin, z. B. hergestellt nach dem deutschen Patent 2 356 493, zu und bestimmt die Zeit, die bis zum Auftreten eines Gerinnsels verstreicht. Zur quantitativen Aussage wird die aus der Faktor VII enthaltenden Lösung sich ergebende Gerinnungszeit unter Bezugnahme auf eine mit einer Normalplasma-Verdünnungsreihe erzielten Eichkurve abgelesen.

Eine Einheit Faktor II entspricht der Faktor-II-Aktivität von 1 ml Normalplasma.

Der Faktor II kann zum Beispiel nach der Methode von Koller, F. et al., Acta haemat. 6, 1 (1951), bestimmt werden. Dazu wird ein Teil, z. B. 0,1 ml Faktor-VII-Mangelplasma und ein Teil verdünntes Normalplasma vermischt. Diese Mischung wird 30 Sekunden bei $+37°$ C gehalten. Danach setzt man 2 Teile calciumhaltiges Thromboplastin, z. B. hergestellt nach dem deutschen Patent 2 356 493, zu und bestimmt die Zeit, die bis zum Auftreten eines Gerinnsels verstreicht. Zur quantitativen Aussage wird die aus der Faktor VII enthaltenden Lösung sich ergebende Gerinnungszeit unter Bezugnahme auf eine mit einer Normalplasma-Verdünnungsreihe erzielten Eichkurve abgelesen.

Eine Einheit Faktor VII entspricht der Faktor-VII-Aktivität von 1 ml Normalplasma.

Zur Abtötung der Hepatitis-Viren werden der Lösung ein Chelatbildner und Glycin und Saccharose zugesetzt und erhitzt.

Für die Weiterreinigung wird die erhitzte Lösung gegebenenfalls zentrifugiert, Verunreinigungen werden durch Ausfällen mit Ammoniumsulfat 30−45% w/v entfernt.

Der Überstand wird an Calciumphosphat, 0,04 bis 1,0% w/v adsorbiert, das beladene Adsorbens gewaschen, mit Citratpuffer eluiert und das Eluat dialysiert.

Für die Anwendung am Menschen wird das Produkt einer Sterilfiltration unterworfen.

Eine nach diesem Verfahren erhältliche proteinarme und hepatitissichere Faktor-II- und -VII-Präparation stellt im besonderen den Gegenstand der Erfindung dar.

Zur Erhöhung der Lagerstabilität ist es zweckmäßig, der Präparation Protein-stabilisierende Substanzen, beispielsweise Proteine, Aminosäuren oder Kohlenhydrate, zuzusetzen. Schließlich kann das dieser Behandlung unterzogene Präparat in gefriergetrockneter Form zur Verfügung gestellt werden, wobei der Zusatz von Antikoagulantien, wie beispielsweise Heparin, vorteilhaft sein kann.

Das erfindungsgemäße Produkt ist, in einer für die pharmazeutische Verabreichung geeigneten Lösung, ein Arzneimittel für die Behandlung von Koagulopathien und kann intravenös, vorteilhaft als Infusion, zur Therapie und Prophylaxe von durch Faktor-II- bzw. Faktor-VII-Mangel bedingten Blutungen eingesetzt werden.

Die Erfindung soll an den nachstehenden Beispielen näher erläutert werden:

**Beispiel 1**

Herstellung eines hepatitissicheren Faktor-II/VII-Konzentrates aus humanem Citratplasma

500 Liter Citratplasma werden mit 250 g Anionenaustauscher (Sephadex-DEAE® Typ A 50) versetzt und 60 Minuten gerührt. Nach Sedimentieren des Adsorbens wird der Plasmaüberstand abgehebert und der Rückstand mit 20 Liter 0,85%iger NaCl-Lösung gewaschen.

Das Adsorbens wird mit 7,5 Liter NaCl-Lösung, 1 mol/l, bei pH 8,0 eluiert und anschließend verworfen. Das Eluat wird mit 1,12 kg Glycin, 11,2 kg Saccharose und 143 g EDTA-Na$_2$ versetzt und bei pH 7,6 10 Stunden bei 60° C erhitzt.

Nach dem Abkühlen wird die Mischung mit 50 Liter dest. Wasser verdünnt und auf eine Ammoniumsulfat-Konzentration von 40% w/v gebracht. Der Niederschlag wird abzentrifugiert und verworfen. Der Überstand wird mit 0,5 kg Ca-Phosphat versetzt und bei pH 7,6 30 Minuten stehengelassen. Nach Zentrifugation wird der Überstand verworfen und das Adsorbens mit je 10 Liter NaCl-Lösung, 0,5 ml/l, zweimal gewaschen. Das Adsorbens wird mit 1,8 Liter Puffer von pH 8,0 eluiert, der 0,2 mol/l Tri-Natrium-Citrat, 0,15 mol/l NaCl, 2 g/100 ml Glycin, 0,3 E/ml Antithrombin III und 14 IE/ml Heparin enthält. Nach Zusatz von 0,2 g/100 ml kolloidaler Kieselsäure als Zentrifugationshilfsmittel wird das Eluat durch Zentrifugation bei 30 000 × g vom Adsorbens abgetrennt. Der Rückstand wird verworfen und der Überstand gegen 100 Liter eines Puffers von pH 7 mit 0,06 mol/l NaCl, 0,02 mol/l Tri-Natrium-Citrat und 2 g/100 ml Glycin 3 Stunden dialysiert. Das Dialysat wird auf Faktor-II- und -VII-Aktivität getestet, auf die gewünschte Konzentration eingestellt, sterilfiltriert, dosiert und lyophylisiert.

Aus 500 Liter Plasma erhält man ca. 250 Abfüllungen zu 200 E Faktor II.

**Beispiel 2**

Erhitzen eines nach dem Verfahren von Soulier et al. (Thromb. Diath. Haemorrh., Suppl. 35, 61, 1969) hergestellten Faktor-II-Komplex-Konzentrates zur Inaktivierung der Hepatitis-Viren

Das Lyophilisat von 4 Abfüllungen Faktor-II-Konzentrat mit jeweils ca. 200 Einheiten wird in 20 ml einer wäßrigen Lösung aufgenommen, die 2,2 mol/l Glycin, 1 g/m Saccharose und 800 mg EDTA-Na$_2$ enthält. Der pH-Wert beträgt 7,6. Nach vollständigem Lösen wird die Abfüllung luftdicht verschlossen und im Wasserbad 10 Stunden bei 60°C inkubiert. Nach Abkühlen wird die Mischung mit 160 ml dest. Wasser verdünnt und auf 40% w/v Ammoniumsulfat-Sättigung gebracht. Der Niederschlag wird abzentrifugiert und der Überstand an 0,8 g Ca-Phosphat adsorbiert.

Alle weiteren Schritte erfolgen entsprechend Beispiel 1 unter Berücksichtigung der übertragbaren Mengenverhältnisse.

## Patentansprüche

1. Verfahren zur Herstellung einer praktisch hepatitissicheren Präparation der Blutgerinnungsfaktoren II und/oder VII durch Erwärmen, in Gegenwart einer Aminosäure und/oder eines Saccharids oder Zuckeralkohols, dadurch gekennzeichnet, daß in Gegenwart eines der Chelatbildner Ethylendiamino-tetraessigsäure (EDTA), Ethylenglycol-bis-(2-aminoethylenether)-tetraessigsäure (EGTA), Diaminocyclohexan-tetraessigsäure (CDTA), Diaminopropan-tetraessigsäure, Diamino-2-propanol-tetraessigsäure oder Nitrilotriessigsäure oder eines ihrer löslichen Alkali-Salze erwärmt wird.

2. Verfahren nach Anspruch 1, dadurch gekennzeichnet, daß der Chelatbildner in einer Konzentration von 0,01 bis 1 mol/l vorliegt.

3. Verfahren nach Anspruch 1, dadurch gekennzeichnet, daß 0,01 bis 0,3 mol/l des Chelatbildners und gegebenenfalls 1,0 bis 3,0 mol/l mindestens einer der Aminosäuren Glycin, alpha- oder $\beta$-Alanin, Hydroxyprolin, Prolin, Glutamin, alpha-, beta- oder gamma-Aminobuttersäure und 20 bis 60% w/w eines Mono-, Oligosaccharids oder Zuckeralkohols zugesetzt und 1 Minute bis 48 Stunden auf eine Temperatur zwischen 30°C und 100°C erhitzt wird.

## Claims

1. Process for the production of a virtually hepatitissafe preparation of blood-clotting Factors II and/or VII by warming in the presence of an aminoacid and/or a saccharide or sugar alcohol, which comprises warming in the presence of one of the chelating agents ethylenediamino-tetraacetic acid (EDTA), ethylene glycole-bis-(2-aminoethyl ether)-tetraacetic acid (EGTA), diaminocyclohexane-tetraacetic acid (CDTA), diaminopropane-tetraacetic acid, diamino-2-propanole-tetraacetic acid or nitrilotriacetic acid or a soluble alkali salt therof.

2. A process as claimed in Claim 1, wherein the chelating agent is present in a concentration of from 0.01 to 1 mole/l.

3. A process as claimed in Claim 1, wherein 0.01 to 0.3 mole/l of the chelating agent and, if appropriate, 1.0 to 3.0 moles/l of at least one of the amino acids glycine, $\alpha$- or $\beta$-alanine, hydroxyproline, proline, glutamine or $\alpha$-, $\beta$- or $\gamma$-aminobutyric acid and 20 to 60% by weight of a monosaccharide, oligosaccharide or sugar alcohole are added and the mixture is heated to a temperature of between 30°C and 100°C for 1 minute to 48 hours.

## Revendications

1. Procédé de fabrication d'une préparation des facteurs de coagulation du sang II et/ou VII pratiquement sûre vis-à-vis de l'hépatite, par chauffage, en présence d'un aminoacide et/ou d'un saccharide ou d'un alcool de sucre, caractérisé en ce qu'on chauffe en présence d'un des chélatants constitués par l'acide éthylènediaminotétracétique (EDTA), l'acide éthylèneglycol-bis (2-aminoéthylène-éther)-tétracétique (EGTA), l'acide diaminocyclohexane-tétracétique (CDTA), l'acide diaminopropane-tétracétique l'acide diamino-2-propanol-tétracétique ou l'acide nitrilotriacétique ou d'un de leurs sels alcalins solubles.

2. Procédé suivant la revendication 1, caractérisé en ce que le chélatant est présent à une concentration de 0,01 à 1 mole/l.

3. Procédé suivant la revendication 1, caractérisé en ce qu'on ajoute 0,01 à 0,3 mole/l du chélatant et, le cas échéant, 1,0 à 3,0 mole/l d'au moins un des aminoacides constitués par la glycine, l'$\alpha$- ou $\beta$-alanine, l'hydroxyproline, la proline, la glutamine, l'acide alpha, béta ou gamma-aminobutyrique et 20 à 60% pds/pds d'un mono-, oligo-saccharide ou d'un alcool de sucre, et on chauffe pendant 1 minute à 48 heures à une température entre 30°C et 100°C.